Europäisches Patentamt

(19) European Patent Office

Office européen des brevets

(11) Numéro de publication : **0 322 277 B1**

(12) # FASCICULE DE BREVET EUROPEEN

(45) Date de publication du fascicule du brevet :
**22.01.92 Bulletin 92/04**

(21) Numéro de dépôt : **88403161.8**

(22) Date de dépôt : **13.12.88**

(51) Int. Cl.⁵ : **A61K 31/55,** A61K 9/20,
A61K 9/22

(54) Formulation pharmaceutique à libération prolongée.

(30) Priorité : **21.12.87 FR 8717855**

(43) Date de publication de la demande :
**28.06.89 Bulletin 89/26**

(45) Mention de la délivrance du brevet :
**22.01.92 Bulletin 92/04**

(84) Etats contractants désignés :
**AT BE CH DE ES FR GB GR IT LI LU NL SE**

(56) Documents cités :
**WO-A-84/02843
US-A- 4 533 674
CHEMICAL ABSTRACTS, vol. 87, no. 22, 28
Novembre 1977, page 308; colonne 2: ref. nO.
189398W; T.STOZEK et al.: "The attempsts of
retarding the release of nitroglycerin from granules & POL. J. PHARMACOL. PHARM 1977, 29
(4), 425-429"
PATENT ABSTRACTS OF JAPAN, vol. 11, no.
184 (C-427) (2631), 12 Juin 1987; & JP-A-62
5915 (NIPPON IYAKUHIN KOGYO K,K.) 12-
01-1987
PATENT ABSTRACTS OF JAPAN, vol. 11, no.
47 (C-403) (2494), 13 Février 1978; JP-A-61
212517 (NIPPON CHEMIPHAR CO.) 20-09-1986**

(73) Titulaire : **SYNTHELABO
58 rue de la Glacière
F-75013 Paris (FR)**

(72) Inventeur : **Stevens, Howard
Ballyconachy Balmaha Road
Drymen G63 0BX Scotland (GB)**
Inventeur : **Chariot, Maryvonne
26, rue Marc Sangnier
F-94700 Maisons- Alfort (FR)**
Inventeur : **Arnold, Françoise
6 Major Lane
Plainsboro New Jersey 08 536 (US)**
Inventeur : **Lewis, Gareth
39, rue de Paris
F-91410 Dourdan (FR)**

(74) Mandataire : **Thouret-Lemaitre, Elisabeth et al
SYNTHELABO Service Brevets 22, avenue
Galilée
F-92352 LE PLESSIS ROBINSON CEDEX (FR)**

EP 0 322 277 B1

## Description

La présente invention a pour objet une formulation pharmaceutique à libération prolongée et sa préparation.

La préparation de formulations pharmaceutiques à libération prolongée est très importante pour l'industrie pharmaceutique : ces formulations permettent au principe actif de se libérer progressivement dans l'organisme et de fournir à ce dernier le principe actif pendant un long moment ; de plus elles évitent au malade l'absorption répétée de comprimés ou gélules.

Plusieurs formulations pharmaceutiques à libération prolongée ont déjà été proposées dans la littérature : comprimés, gélules de micrograins enrobés.

La présente invention a pour objet des formulations pharmaceutiques à libération prolongée qui permettent une dissolution contrôlée du principe actif, pendant une longue période de temps, indépendamment du pH.

Les formulations pharmaceutiques de l'invention sont constituées par des micrograins contenant le principe actif et, éventuellement, des excipients, et enrobés par un mélange de polymères, les micrograins étant ensuite introduits dans une gélule.

Le mélange de polymères constituant l'enrobage est caractéristique de l'invention : c'est un mélange d'éthylcellulose et d'Eudragit ®, plus spécifiquement un mélange d'éthylcellulose et d'Eudragit RS dans des proportions allant de 60/40 à 40/60.

Les micrograins non enrobés sont constitués par un mélange du principe actif, d'un diluant et d'un liant.

Le principe actif peut constituer de 40 à 99% en poids du micrograin, plus particulièrement 80%.

Le diluant est, par exemple, la cellulose microcristalline. Le liant est, par exemple, la polyvinylpyrrolidone, la méthylhydroxypropylcellulose et, de préférence, la carboxyméthylcellulose.

L'enrobage du micrograin est constitué par un mélange de polymères qui peut, en plus, contenir un plastifiant et est appliqué aux micrograins dans un solvant ou un mélange de solvants.

Le mélange de polymères qui permet la dissolution contrôlée, indépendante du pH, du principe actif est un mélange d'éthylcellulose (40 à 60% en poids) et d'Eudragit RS (60 à 40% en poids).

L'Eudragit RS est une résine acrylique, polymérisat d'esters d'acides acrylique et méthacrylique, fabriquée par Röhm Pharma GmbH.

Ce produit est insoluble dans l'eau, les sucs gastrointestinaux naturels et artificiels et les solutions tamponnées, mais il gonfle et devient perméable dans ces liquides.

Le mélange d'enrobage contient de préférence 45% d'éthylcellulose et 55% d'Eudragit RS.

Le plastifiant peut être le phtalate de diéthyle, le phtalate de dibutyle, un monoglycéride acétylé, du propylèneglycol, le sébaçate de dibutyle, le triacétate de glycérol, un ester d'acide citrique tel que le citrate de triéthyle, l'acétylcitrate de triéthyle, le citrate de tributyle, l'acétylcitrate de tributyle, l'acétyl-citrate de tri-(éthyl-2 hexyle). On utilise de préférence un monoglycéride acétylé.

Selon les proportions des deux constituants du mélange et l'épaisseur du film d'enrobage la libération du principe actif peut être modulée.

Les solvants utilisés pour appliquer l'enrobage sur les micrograins sont l'eau ou, de préférence, des solvants organiques tels que l'acétone, l'acétate d'éthyle, le chlorure de méthylène, l'alcool isopropylique.

On peut également utiliser un mélange de solvants, tel qu'un mélange d'alcool isopropylique et d'acétone dans des proportions de 10/90 à 90/10.

Le mélange d'enrobage est contenu dans le solvant ou le mélange de solvants à raison de 4 à 8%.

Selon l'invention, on prépare les formulations pharmaceutiques en deux temps : on fabrique d'abord les micrograins puis on les enrobe.

La fabrication des micrograins peut être effectuée selon différentes méthodes :
— montage traditionnel
— rotogranulation
— compactage
— extrusion-sphéronisation.

La dernière méthode est la méthode préférée.

L'enrobage est appliqué par pulvérisation à l'aide d'un appareil d'enrobage qui peut être une turbine traditionnelle, une turbine ventilée, un lit d'air fluidisé (pulvérisation en "Top-Spray" ou "Bottom-Spray" avec ou sans colonne) ou un rotogranulateur (pulvérisation tangentielle).

Selon l'invention, on réalise de préférence l'enrobage en lit d'air fluidisé, par pulvérisation "Bottom-Spray" avec colonne.

Les formulations pharmaceutiques à libération prolongée de l'invention peuvent contenir divers principes actifs et en particulier du diltiazem.

Un exemple de formulation est le suivant

- <u>Micrograins</u>

| | |
|---|---|
| Chlorhydrate de diltiazem | 80% en poids |
| Cellulose microcristalline | 19% " |
| Carboxyméthylcellulose | 1% " |

- <u>Enrobage</u>

| | |
|---|---|
| Ethylcellulose N 22 NF | 41% en poids |
| Eudragit RS | 50% " |
| Monoglycéride acétylé Myvacet 9-40 | 9% " |

sous la forme d'une solution à 6% dans un mélange 65/35 d'acétone/alcool isopropylique que l'on pulvérise jusqu'à ce que l'enrobage représente environ 4% du poids sec du micrograin.

Les formulations à libération prolongée de l'invention, sont constituées par des micrograins enrobés contenant de 70 à 80% de principe actif. Dans ces conditions les gélules peuvent contenir de 90 à 400 mg de principe actif.

La Demanderesse a fait des études comparatives de dissolution de micrograins enrobés différemment ou non

— la dissolution de micrograins non enrobés (ne contenant que le principe actif, en particulier du diltiazem) est dépendante du pH,

— la dissolution de micrograins enrobés par de l'éthylcellulose est dépendante du pH,

— la dissolution des micrograins enrobés par de l'Eudragit RS est dépendante du pH,

— la dissolution de micrograins enrobés par un mélange éthylcellulose/Eudragit RS, dans les proportions retenues 40/60 à 60/40, est indépendante du pH.

Le fait que la dissolution des formulations pharmaceutiques à libération prolongée de l'invention soit indépendante du pH est très important : la libération du principe actif est indépendante du milieu tout au long du tractus gastrointestinal et peut se faire régulièrement.

## Revendications

**Revendications pour les Etats contractants suivants : AT, BE, CH, DE, FR, GB, IT, LI, LU, NL, SE.**

1. Formulation pharmaceutique à libération prolongée permettant la dissolution contrôlée du principe actif, indépendamment du pH, caractérisée par le fait qu'elle consiste en micrograins contenant le principe actif enrobés par un mélange constitué par 60 à 40% en poids d'éthylcellulose et 40 à 60% en poids d'Eudragit RS.

2. Formulation pharmaceutique selon la revendication 1 caractérisée par le fait que le mélange d'enrobage est constitué par 45% en poids d'éthylcellulose et 55% en poids d'Eudragit RS.

3. Formulation pharmaceutique selon l'une quelconque des revendications 1 et 2, caractérisée par le fait que le mélange d'enrobage contient également un plastifiant.

4. Formulation pharmaceutique selon la revendication 3, caractérisée par le fait que le mélange d'enrobage contient un monoglycéride acétylé.

5. Formulation pharmaceutique selon l'une quelconque des revendications 1 à 4, caractérisée par le fait que les micrograins contiennent le principe actif en association avec un diluant et un liant.

6. Formulation pharmaceutique selon l'une quelconque des revendications 1 à 5, caractérisée par le fait que le principe actif est le chlorhydrate de diltiazem.

7. Formulation pharmaceutique selon la revendication 6, caractérisée par le fait que le diluant est la cellulose microcristalline.

8. Formulation pharmaceutique selon la revendication 6, caractérisée par le fait que le liant est la carboxyméthylcellulose.

**Revendications pour les Etats contractants suivants : ES et Gr**

1. Procédé de préparation d'une formulation pharmaceutique à libération prolongée permettant la dissolution contrôlée du principe actif, indépendamment du pH, caractérisée par le fait que l'on enrobe des micrograins contenant le principe actif par un mélange constitué par 60 à 40% en poids d'éthylcellulose et 40 à 60% en poids d'Eudragit RS.

2. Procédé selon la revendication 1 caractérisé par le fait que le mélange d'enrobage est constitué par 45% en poids d'éthylcellulose et 55% en poids d'Eudragit RS.

3. Procédé selon l'une quelconque des revendications 1 et 2, caractérisé par le fait que le mélange d'enrobage contient également un plastifiant.

4. Procédé selon la revendication 3, caractérisé par le fait que le mélange d'enrobage contient un monoglycéride acétylé.

5. Procédé selon l'une quelconque des revendications 1 à 4, caractérisé par le fait que les micrograins contiennent le principe actif en association avec un diluant et un liant.

6. Procédé selon l'une quelconque des revendications 1 à 5, caractérisé par le fait que le principe actif est le chlorhydrate de diltiazem.

7. Procédé selon la revendication 6, caractérisé par le fait que le diluant est la cellulose microcristalline.

8. Procédé selon la revendication 6, caractérisé par le fait que le liant est la carboxyméthylcellulose.

## Patentansprüche

### Patentansprüche für folgende Vertragsstaaten : AT, BE, CH, DE, FR, GB, IT, LI, LU, NL, SE

1. Pharmazeutische Zusammensetzung mit verzögerter Wirkstoffabgabe, die eine kontrollierte, pH-unabhängige Auflösung des Wirkstoffs gestattet, dadurch gekennzeichnet, daß sie in Form von Kügelchen vorliegt, die den Wirdstoff enthalten und eingehüllt von einer Mischung aus 60 bis 40 Gew.-% Ethylcellulose und 40 bis 60 Gew.-% Eudragit RS sind.

2. Pharmazeutische Zusammensetzung nach Anspruch 1, dadurch gekennzeichnet, daß die Hüllmischung aus 45 Gew.-% Ethylcellulose und 55 Gew.-% Eudragit RS besteht.

3. Pharmazeutische Zusammensetzung nach Anspruch 1 oder 2, dadurch gekennzeichnet, daß die Hüllmischung auch einen Weichmacher enthält.

4. Pharmazeutische Zusammensetzung nach Anspruch 3, dadurch gekennzeichnet, daß die Hüllmischung ein acetyliertes Monoglycerid enthält.

5. Pharmazeutische Zusammensetzung nach einem der Ansprüche 1 bis 4, dadurch gekennzeichnet, daß die Kügelchen den Wirkstoff in Kombination mit einem Verdünnungs- und einem Bindemittel enthalten.

6. Pharmazeutische Zusammensetzung nach einem der Ansprüche 1 bis 5, dadurch gekennzeichnet, daß der Wirkstoff Diltiazem-Chlorhydrat ist.

7. Pharmazeutische Zusammensetzung nach Anspruch 6, dadurch gekennzeichnet, daß das Verdünnungsmittel mikrokristalline Cellulose ist.

8. Pharmazeutische Zusammensetzung nach Anspruch 6, dadurch gekennzeichnet, daß das Bindemittel Carboxymethylcellulose ist.

### Patentansprüche für folgende Vertragsstaaten : ES und GR

1. Verfahren zur Herstellung einer pharmazeutischen Zusammensetzung mit verzögerter Wirkstoffabgabe, die eine kontrollierte, pH-unabhängige Auflösung des Wirkstoffs gestattet, dadurch gekennzeichnet, daß man wirkstoffhaltige Kügelchen mit einer Mischung aus 60 bis 40 Gew.-% Ethylcellulose und 40 bis 60 Gew.-% Eudragit RS umhüllt.

2. Verfahren nach Anspruch 1, dadurch gekennzeichnet, daß die Hüllmischung aus 45 Gew.-% Ethylcellulose und 55 Gew.-% Eudragit RS besteht.

3. Verfahren nach Anspruch 1 oder 2, dadurch gekennzeichnet, daß die Hüllmischung auch einen Weichmacher enthält.

4. Verfahren nach Anspruch 3, dadurch gekennzeichnet, daß die Hüllmischung ein acetyliertes Monoglycerid enthält.

5. Verfahren nach einem der Ansprüche 1 bis 4, dadurch gekennzeichnet, daß die Kügelchen den Wirkstoff in Kombination mit einem Verdünnungs- und einem Bindemittel enthalten.

6. Verfahren nach einem der Ansprüche 1 bis 5, dadurch gekennzeichnet, daß der Wirkstoff Diltiazem-Chlorhydrat ist.

7. Verfahren nach Anspruch 6, dadurch gekennzeichnet, daß das Verdünnungsmittel mikrokristalline Cellulose ist.

8. Verfahren nach Anspruch 6, dadurch gekennzeichnet, daß das Bindemittel Carboxymethylcellulose ist.

**Claims**

**Claims for the following Contracting States : AT, BE, CH, DE, FR, GB, IT, LI, LU, NL, SE.**

1. Sustained-release pharmaceutical dosage form permitting the controlled dissolution of the active principle, independently of the pH, characterized in that it consists of microparticles containing the active principle and coated with a mixture consisting of 60 to 40% by weight of ethylcellulose and 40 to 60% by weight of Eudragit RS.

2. Pharmaceutical dosage form according to Claim 1, characterized in that the coating mixture consists of 45% by weight of ethylcellulose and 55% by weight of Eudragit RS.

3. Pharmaceutical dosage form according to either one of Claims 1 and 2, characterized in that the coating mixture also contains a plasticizer.

4. Pharmaceutical dosage form according to Claim 3, characterized in that the coating mixture contains an acetylated monoglyceride.

5. Pharmaceutical dosage form according to any one of Claims 1 to 4, characterized in that the microparticles contain the active principle in combination with a diluent and a binder.

6. Pharmaceutical dosage form according to any one of Claims 1 to 5, characterized in that the active principle is diltiazem hydrochloride.

7. Pharmaceutical dosage form according to Claim 6, characterized in that the diluent is microcrystalline cellulose.

8. Pharmaceutical dosage form according to Claim 6, characterized in that the binder is carboxymethylcellulose.

**Claims for the following Contracting States : ES and GR.**

1. Process for the preparation of a sustainedrelease pharmaceutical dosage form permitting the controlled dissolution of the active principle, independently of the pH, characterized in that microparticles containing the active principle are coated with a mixture consisting of 60 to 40% by weight of ethylcellulose and 40 to 60% by weight of Eudragit RS.

2. Process according to Claim 1, characterized in that the coating mixture consists of 45% by weight of ethylcellulose and 55% by weight of Eudragit RS.

3. Process according to either of Claims 1 and 2, characterized in that the coating mixture also contains a plasticizer.

4. Process according to Claim 3, characterized in that the coating mixture contains an acetylated monoglyceride.

5. Process according to any one of Claims 1 to 4, characterized in that the microparticles contain the active principle in combination with a diluent and a binder.

6. Process according to any one of Claims 1 to 5, characterized in that the active principle is diltiazem hydrochloride.

7. Process according to Claim 6, characterized in that the diluent is microcrystalline cellulose.

8. Process according to Claim 6, characterized in that the binder is carbomethylcellulose.